# EUROPEAN PATENT APPLICATION

(11) **EP 4 378 401 A1**
(43) Date of publication of application: **05.06.2024**
(21) Application number: 21959084.1
(22) Date of filing: 02.11.2021
(51) Int. Cl.: A61B 17/12

(54) **NECK ANEURYSM BLOCKING DEVICE**

(30) Priority: 28.09.2021 CN 202111146298
(71) Applicant: Beijing Taijieweiye Technology Co., Ltd., Beijing 101204 (CN)
(72) Inventor: MU, Lei, Beijing 101204 (CN); CHENG, Wenjie, Beijing 101204 (CN); XU, Yongsong, Beijing 101204 (CN); QIN, Chuan, Beijing 101204 (CN); TANG, Hang, Beijing 101204 (CN); ZHANG, Ting, Beijing 101204 (CN); LIAO, Zhimei, Beijing 101204 (CN); WANG, Jin, Beijing 101204 (CN); WANG, Xiaoting, Beijing 101204 (CN)
(74) Representative: Glück Kritzenberger Patentanwälte PartGmbB
(86) International application number: PCT/CN2021/128044
(87) International publication number: WO 2023/050520

(57) **Abstract**

The present disclosure relates to a neck aneurysm blocking device, comprising a plurality of layers of structures attached to each another. At least one structure has the shape of a metal mesh disc; the metal mesh disc is formed by interweaving metal wires; and the centers of the plurality of layers of structures are fixedly connected by means of a developing mark. During use, the sparser mesh discs of the plurality of layers of structures can be attached to an inner wall of an arterial aneurysm, and the denser metal mesh discs are fixed to a neck aneurysm, such that the blood flow in an aneurysm cavity is blocked, a stimulus to a blood vessel is reduced to the utmost extent, and complications are effectively reduced.

## Description

The application claims the priority to Chinese Patent Application No. 202111146298.3 filed with the Patent Office of the People's Republic of China on September 28, 2021 and entitled "Neck Aneurysm Blocking Device" in.

### BACKGROUND OF THE INVENTION

### 1. Technical Field

The present disclosure relates to the field of medical instruments, and particularly relates to a neck aneurysm blocking device.

### 2. Description of Related Art

Arterial aneurysm refers to persistent dilation of a localized arterial segment caused by lesion or damage on a side wall of the arterial aneurysm. The detection rate of the intracranial arterial aneurysm in groups in China reaches about 9%, and the incidence rate in the groups with intracranial arterial aneurysm is about 3.2%.The intracranial arterial aneurysm is classified into narrow neck (<4mm) and wide neck (neck aneurysm≥4mm or body-neck ratio<2) according to the size of neck aneurysm.

Treatment for the intracranial arterial aneurysm includes interventional therapy, surgical clipping, conservative treatment and the like. With the increasing development of interventional radiology and the emergence of novel materials and technologies in recent years, interventional therapy in treatment of intracranial aneurysm has occupied an irreplaceable position and has the trend of gradually replacing surgical operations.

A spring coil is a significant milestone in development of interventional therapy for intracranial aneurysm. However, due to a risk of escape of the spring coil and a high recurrence rate of wide-necked aneurysm, it is still a problem for treating wide-necked aneurysm by means of the pure spring coil. With the emergence of various stents and balloon assisted technologies, the spring coil embolization technology is increasingly perfected, and its curative effect and prognosis are verified sufficiently. The spring coil embolization technology still has the following inherent defects: first of all, it has a higher requirement for operation of a surgeon; as the effect of treating intracranial aneurysm by means of the spring coil is related to the compact embolization degree thereof, the surgeon is asked to place the spring coils as many as possible in the surgical procedure, striving for compact embolization; then, it causes the higher recurrence rate of aneurysm, and in a long-term clinical follow-up, recanalization occurs on 1/3 of patients; with respect to patients with fully embolized aneurysm, recanalization of aneurysm happens to over 26.4% of patients; third, the spring coil has a permanent mass effect; and finally, treatment of aneurysm by means of spring coil embolization has a higher bleeding risk due to delayed crack of aneurysm.

To overcome these defects of the spring coil in treating intracranial aneurysm by means, a concept of re-building a parent artery is put forward, so that a blood flow guiding device is born at the right moment. The blood flow guiding device is a stent with a high metal surface coverage rate and is unnecessary to enter an aneurysm cavity. The stent is placed in the parent artery to re-build the patent artery in haemodynamics so as to induce spontaneous thrombosis in the aneurysm. The inner surface of a vessel lumen is re-shaped through a new endangium on the neck surface of aneurysm, so as to finally achieve the object of curing aneurysm. In recent years, the blood flow guiding device has achieved unprecedented development. However, in its using process, there are still some defects hard to overcome: first, after the blood flow guiding device is implanted, the patient needs to take antiplatelet drugs for a long time, which brings a hidden danger to intracranial hemorrhage; second, a branch vessel is covered to lead to occlusion, and particularly, narrow blood vessel occlusion complications are likely to happen in treating wide-necked aneurysm at a furcation; third, intraluminal thrombi of the blood flow guiding device are formed, which is one of the most severe complications in use of the blood flow guiding device, and it is necessary to perform dual-antibody treatment before and after the operation; and fourth, the blood flow guiding device has a high requirement for operation by the surgeon, and is extremely like to open in a complex tortuous blood vessel with poor effect, which causes hemadostenosis and even occlusion.

In addition, it is still quite difficult to perform intravascular interventional therapy for wide-necked aneurysm at the furcation of an intracranial artery, which approximately accounts for 2.9%-7.1% of all intracranial aneurysms. With respect to the intravascular therapy for wide-necked aneurysm at the furcation of the blood vessel and other wide-necked aneurysms, the most significant problem lies in that stable plugging of the spring coils, the stent assisted spring coils and the blood flow guiding device affect the branch vessel and relapse of aneurysm.

In recent years, several types of novel devices for treating aneurysm, i.e., intratumoral turbulence devices, have been developed. This type of devices has the following main features: first, they have the advantage of being free of antiplatelet therapy after the operation; and second, they are more suitable for wide-necked aneurysm, particularly aneurysm at the furcation, which is an important supplement for treating aneurysm by means of the blood flow guiding device.

The intratumoral turbulence devices represented by Woven EndoBridge (WEB) are similar to spring coil embolization, and are also implanted into the cavity of arterial aneurysm to play a blood rebuilding role without affecting the parent artery. Various intratumoral turbulence devices such as WEB, Artisse Device and Medina have been developed at present. However, WEB and Artisse Device are integrally spherical with a high model selection difficulty when they are used for filling arterial aneurysm and still have the problems of mass effect and residues at the aneurysm neck similar to the spring coil. The filling mode of the Medina device is similar to use of the spring coil, with obvious mass effect and residues at the aneurysm neck.

With respect to the intratumoral turbulence devices represented by Contour Neurovascular System (CNS), the CNS device is integrally disc-like. Although it can reduce the intratumoral mass effect after being implemented into the cavity of arterial aneurysm, the proximal mark protrudes prominently when it is placed in the arterial aneurysm, so that it is easy to induce thrombotic events in the vessel lumen; moreover, it has few specifications, thus being unable to cope with various arterial aneurysms with varied dimensions: a double-layered weaving structure obtained by directly turning the device features high rigidity, is hard to comply with the shape of the arterial aneurysm and is likely to induce shape change of the arterial aneurysm after being implanted, thereby resulting in bleeding and rupturing risks; the most significant problem thereof lies in poor stability and displacement thereof after being implanted with impact of blood flow, which causes a failure of arterial aneurysm embolization.

### BRIEF SUMMARY OF THE INVENTION

To overcome the defects in the prior art, the object of the present disclosure is to provide a neck aneurysm blocking device to at least partially solve the above technical problems.

To achieve the above object, the present disclosure provides a neck aneurysm blocking device, including a multi-layered structure with mutually attached layers, where at least one of the layers has a shape of a metal mesh disc;
the metal mesh disc is formed by interweaving metal wires; and
centers of respective layers in the multi-layered structure are fixedly connected by means of a developing mark.

In some solutions, the multi-layered structure includes two layers of structures including a first metal mesh disc and a second metal mesh disc.

In some solutions, the first metal mesh disc and the second metal mesh disc are interwoven together through the metal wires.

In some solutions, a weaving density of the metal wires of the second metal mesh disc is greater than a weaving density of the metal wires of the first metal mesh disc.

In some solutions, an outer diameter of the second metal mesh disc is smaller than an outer diameter of the first metal mesh disc.

In some solutions, a height of the developing mark is 5%-40% of the radius of the first metal mesh disc.

In some solutions, a bottom end of the developing mark does not exceed the lowest end of the multi-layered structure.

In some solutions, the metal mesh disc has a multi-petal structure.

In some solutions, a number of the metal wires of the metal mesh disc is 20-200.

In some solutions, a wire diameter of the metal wires of the metal mesh disc is 0.01-0.07 mm.

In some solutions, the metal wires of the metal mesh disc are nickel-titanium alloy wires, nickel-titanium wires including 10%-30% of platinum, cobalt-chromium alloy wires including 10%-30% of platinum or 35 NLT alloy wires.

In some solutions, the developing mark is made from gold, tantalum, a platinum-iridium alloy or a platinum-tungsten alloy.

The embodiment of the present disclosure provides a neck aneurysm blocking device. The blocking device having the multi-layered structure includes a plurality of layers of metal mesh discs with different weaving densities, where the larger metal mesh disc is smaller in weaving density and larger in mesh, and plays a role of fixing the device in a cavity of an arterial aneurysm; the smaller metal mesh disc is higher in density and smaller in mesh, and can block a blood flood in the cavity of the arterial aneurysm; the metal mesh disc is formed by weaving the metal wires, is integrally soft and flexible and high in compliance, and can be adaptive to shapes of the cavity and neck of the arterial aneurysm to achieve embolization at the neck of the aneurysm without affecting the parent artery; thus, it reduces stimulation to the blood vessel when foreign matters are imported into the cavity of the arterial aneurysm to the maximum extent while efficiently blocking the blood flow in the cavity of the aneurysm, thereby effectively reducing complications.

### BRIEF DESCRIPTION OF THE SEVERAL VIEWS OF THE DRAWINGS

FIG. 1 is a schematic diagram of a top view of a neck aneurysm blocking device according to an embodiment of the present disclosure;
FIG. 2 is a schematic diagram of a side view of a neck aneurysm blocking device according to an embodiment of the present disclosure;
FIG. 3 is a schematic diagram of a conveying state of a neck aneurysm blocking device according to an embodiment of the present disclosure;
FIG. 4 is a working schematic diagram of a neck aneurysm blocking device in an arterial aneurysm according to an embodiment of the present disclosure;
FIG. 5 is a structural schematic diagram of a metal wire of a metal mesh disc according to an embodiment of the present disclosure;
FIG. 6 is a schematic diagram of a weaving mode of the metal wire according to an embodiment of the present disclosure; and
FIG. 7 is a schematic diagram of a side view of a neck aneurysm blocking device according to an embodiment of the present disclosure.

### DETAILED DESCRIPTION OF THE INVENTION

The technical solution of the present disclosure will be further described in detail below in combination with drawings and embodiments.

The embodiment of the present disclosure provides a neck aneurysm blocking device, including a multi-layered structure attached to each another. At least one structure has the shape of a metal mesh disc; the metal mesh disc is formed by interweaving metal wires; and the centers of the multi-layered structure are fixedly connected together by means of a developing mark.

The present disclosure is described in detail below through specific embodiments. It should be understood that the embodiments below are not intended to limit the present disclosure. Those skilled in the art are capable of thinking of arranging and combining specific features in the embodiments to form other similar solutions based on the concept of the present disclosure.

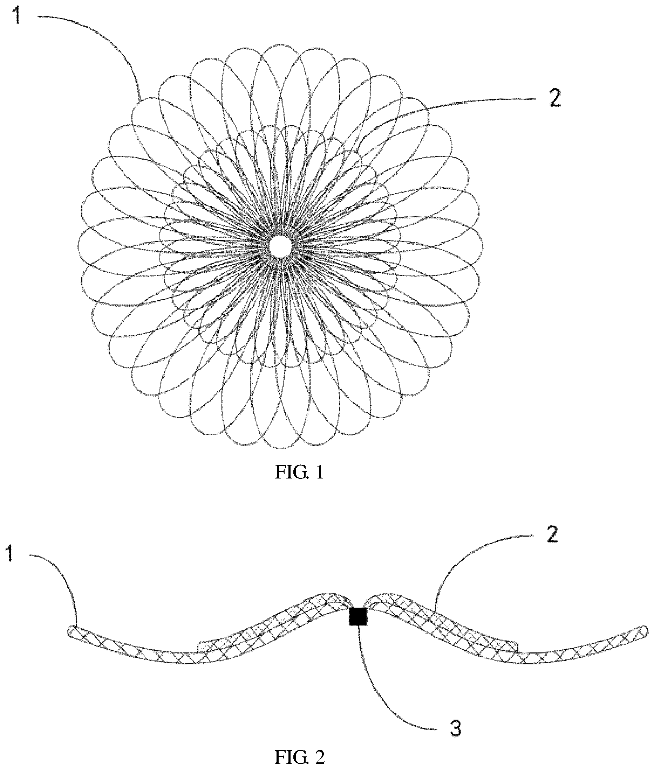

FIG. 1 is a schematic diagram of a top view of a neck aneurysm blocking device according to an embodiment of the present disclosure; and FIG. 2 is a schematic diagram of a side view of a neck aneurysm blocking device according to an embodiment of the present disclosure. As shown in FIG. 1 and FIG. 2, the neck aneurysm blocking device includes a multi-layered structure consisting of a first metal mesh disc 1 and a second metal mesh disc 2, and a developing mark 3.

The multi-layered structure of the neck aneurysm blocking device according to the embodiment of the present disclosure includes the first metal mesh disc 1 and the second metal mesh disc 2 attached to each other, where the first metal mesh disc 1 and the second metal mesh disc 2 are metal mesh discs with different sizes. The metal mesh discs are formed by interweaving metal wires and are disc-like in an unfolded state; an outer diameter of the second metal mesh disc 2 is smaller than that of the first metal mesh disc 1. Attached to each other means that one side of the second metal mesh disc 2 tightly contacts one side of the first metal mesh disc 1, centers of respective layers in the multi-layered structure, i.e., a center of the first metal mesh disc 1 and a center of the second metal mesh disc 2, are fixed in the developing mark 3.

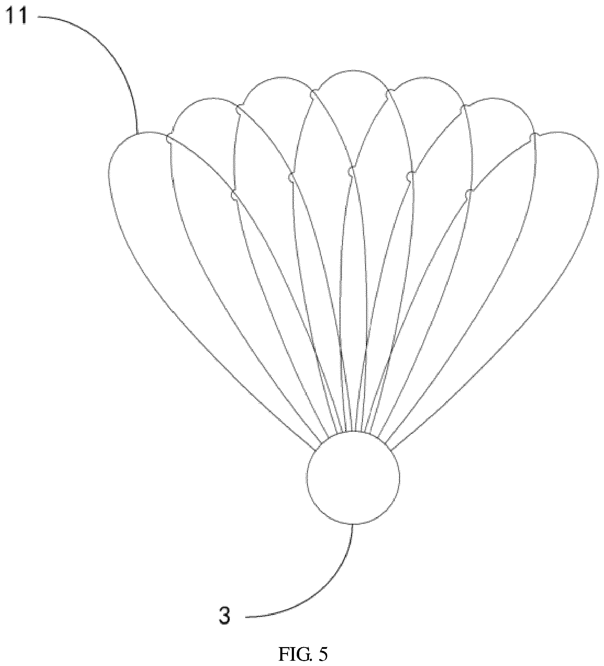

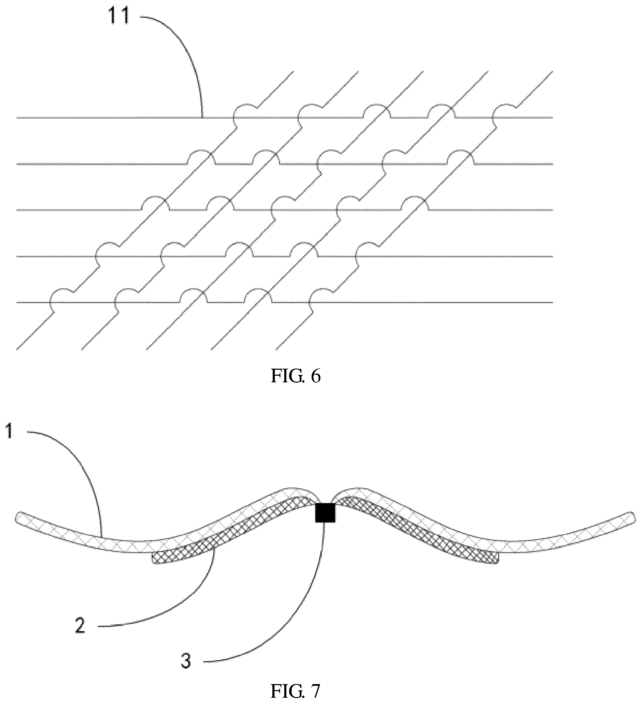

FIG. 5 is a structural schematic diagram of a metal wire of a metal mesh disc according to an embodiment of the present disclosure. As shown in FIG. 5, the first metal mesh disc 1 and the second metal mesh disc 2 are both the metal mesh discs formed by interweaving the metal wires 11. Specifically, to better achieve a blocking effect, the number of the metal wires 11 used in each metal mesh disc can be 20-200, both ends of each metal wire 11 are fixed in the developing mark 3, and the metal wires 11 are naturally bent in petal structures. Therefore, the metal mesh disc formed by a plurality of petal structures is integrally petal-shaped, the plurality of metal wires 11 are interwoven at an interval, and the plurality of metal wires 11 are not fixed and can slide relatively. As shown in FIG. 6, the number of the interwoven metal wires 11 at an interval can be two or more, and the compliance of the metal mesh discs can be correspondingly adjusted by adjusting the number of the interwoven metal wires 11 at an interval, so that the metal mesh discs can be better attached to the inner wall and the neck aneurysm in the aneurysm cavity.

The metal wires of the first metal mesh disc 1 and the metal wires of the second metal mesh disc 2 can also be interwoven in the above manner, so that the first metal mesh disc 1 and the second metal mesh disc 2 can be tightly attached together.

The length of each of the metal wires 11 of the first metal mesh disc 1 is smaller than that of the metal wire 11 of the second metal mesh disc 2, and the outer diameter of the second metal mesh disc 2 formed by weaving the metal wires is smaller than that of the first metal mesh disc 1.

The metal wires 11 of the first metal mesh disc 1 are smaller in weaving density and larger in mesh, and plays a role of fixing the device in a cavity of an arterial aneurysm; a weaving density of the metal wires 11 of the second metal mesh disc 2 is greater than that of the metal wires 11 of the first metal mesh disc 1, and the metal wires of the second metal mesh disc 2 are greater in weaving density and smaller in mesh, thereby blocking the blood flood in the cavity of the arterial aneurysm.

The wire diameter of the metal wires 11 of the metal mesh disc can be 0.01-0.07 mm. The metal wires 11 of the metal mesh disc may be nickel-titanium alloy wires, nickel-titanium wires including 10%-30% of platinum, cobalt-chromium alloy wires including 10%-30% of platinum or 35 NLT alloy wires.

The developing mark 3 is an X- ray radiopaque mark, and the developing mark 3 can be made from gold, tantalum, a platinum-iridium alloy or a platinum-tungsten alloy. To prevent the blood flow in the blood vessel from being affected as the developing mark 3 protrudes out of the aneurysm neck, the height of the developing mark 3 is set to be 5%-40% of the radius of the first metal mesh disc 1. In such a manner, when the device is in a fully unfolded state, the bottom end of the developing mark 3 does not exceed the lowest end of the multi-layered structure, so that the blood flow in the blood vessel will not be affected.

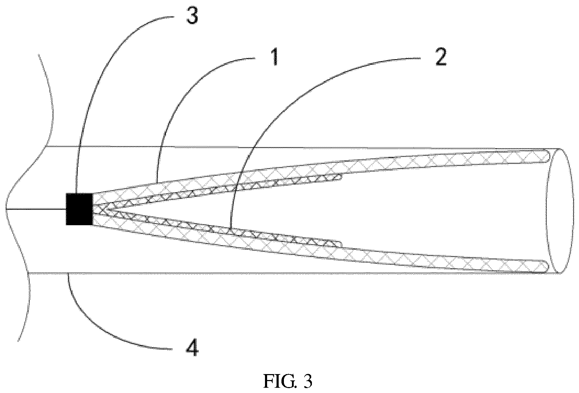

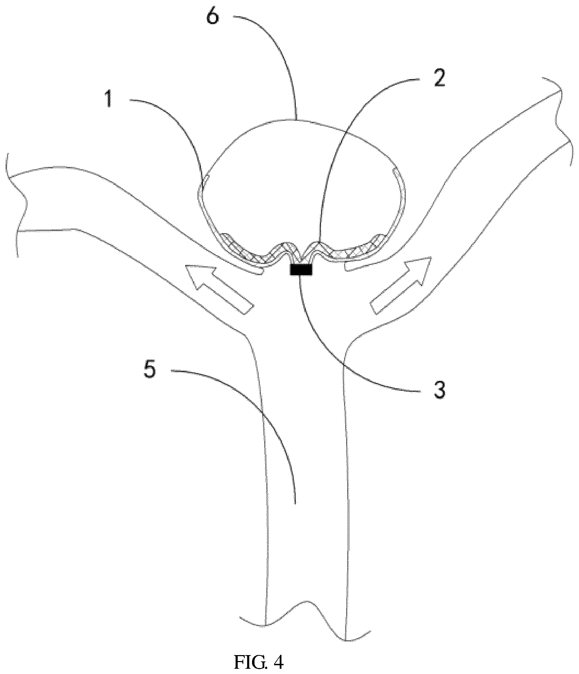

FIG. 3 is a schematic diagram of a conveying state of a neck aneurysm blocking device according to an embodiment of the present disclosure; and FIG. 4 is a working schematic diagram of a neck aneurysm blocking device according to an embodiment of the present disclosure in an arterial aneurysm. As shown in FIG. 3 and FIG. 4, during use, the neck aneurysm blocking device is conveyed by the conveying conduit 4; in the conveying conduit 4, the neck aneurysm blocking device is compressed into a bundle; when the conveying conduit 4 conveys the neck aneurysm blocking device to the neck aneurysm of the arterial aneurysm 6, the neck aneurysm blocking device is pushed to the conveying conduit 4 through a guiding wire and is sent into the arterial aneurysm 6, the neck aneurysm blocking device is unfolded in the arterial aneurysm 6, the first metal mesh disc 1 thereof is attached to the inner wall of the arterial aneurysm 6, the second metal mesh disc 2 blocks the neck aneurysm, and meanwhile, the developing mark 3 retracts into the neck aneurysm and is prevented from protruding in the blood vessel 5 to induce thrombi.

FIG. 7 is a schematic diagram of a side view of a neck aneurysm blocking device according to another embodiment of the present disclosure. As shown in FIG. 7, the second metal mesh disc 2 can further be located below the first metal mesh disc 1, and the working principle and achieved technical effects thereof both are the same as those in the aforementioned embodiment.

The embodiment of the present disclosure provides a neck aneurysm blocking device. The blocking device having the multi-layered structure includes a plurality of layers of metal mesh discs with different weaving densities, where the larger metal mesh disc is smaller in weaving density and larger in mesh, and plays a role of fixing the device in a cavity of an arterial aneurysm; the smaller metal mesh disc is higher in density and smaller in mesh, and can block a blood flood in the cavity of the arterial aneurysm; the metal mesh disc is formed by weaving the metal wires, is integrally soft and flexible and high in compliance, and can be adaptive to shapes of the cavity and neck of the arterial aneurysm to achieve embolization at the aneurysm neck without affecting the parent artery; thus, it reduces stimulation to the blood vessel when foreign matters are imported into the cavity of the arterial aneurysm to the maximum extent while efficiently blocking the blood flow in the cavity of the aneurysm, thereby effectively reducing complications.

The objects, technical solutions and beneficial effects of the present disclosure are further described in detail in the above specific implementation modes. It shall be understood that the above is merely the specific implementations of the present disclosure and is not intended to limit the protection scope of the present disclosure. Any modification, equivalent substitution, improvement and the like made within the spirit and principle of the present disclosure shall be included in the protection scope of the present disclosure.

## Claims

1. A neck aneurysm blocking device, comprising a multi-layered structure with mutually attached layers, wherein at least one of the layers has a shape of a metal mesh disc;
the metal mesh disc is formed by interweaving metal wires; and
centers of respective layers in the multi-layered structure are fixedly connected by means of a developing mark.

2. The neck aneurysm blocking device according to claim 1, wherein the multi-layered structure comprises two layers comprising a first metal mesh disc and a second metal mesh disc.

3. The neck aneurysm blocking device according to claim 2, wherein the first metal mesh disc and the second metal mesh disc are interwoven together through the metal wires.

4. The neck aneurysm blocking device according to claim 2, wherein a weaving density of the metal wires of the second metal mesh disc is greater than a weaving density of the metal wires of the first metal mesh disc.

5. The neck aneurysm blocking device according to claim 2, wherein an outer diameter of the second metal mesh disc is smaller than an outer diameter of the first metal mesh disc.

6. The neck aneurysm blocking device according to claim 2, wherein a height of the developing mark is 5%-40% of a radius of the first metal mesh disc.

7. The neck aneurysm blocking device according to claim 1, wherein a bottom end of the developing mark does not exceed a lowest end of the multi-layered structure.

8. The neck aneurysm blocking device according to claim 1, wherein the metal mesh disc has a multi-petal structure.

9. The neck aneurysm blocking device according to claim 1, wherein a number of the metal wires of the metal mesh disc is 20-200.

10. The neck aneurysm blocking device according to claim 1, wherein a wire diameter of the metal wires of the metal mesh disc is 0.01-0.07 mm.

11. The neck aneurysm blocking device according to claim 1, wherein the metal wires of the metal mesh disc are nickel-titanium alloy wires, nickel-titanium wires comprising 10%-30% of platinum, cobalt-chromium alloy wires comprising 10%-30% of platinum or 35 NLT alloy wires.

12. The neck aneurysm blocking device according to claim 1, wherein the developing mark is made from gold, tantalum, a platinum-iridium alloy or a platinum-tungsten alloy.
